# FASCICULE DE BREVET EUROPEEN

(11) **EP 3 019 020 B1**
(45) Date de publication et mention de la délivrance du brevet: **09.08.2017**
(21) Numéro de dépôt: 14738905.0
(22) Date de dépôt: 20.06.2014
(51) Int. Cl.: A23D 9/00, A61K 31/00, C11C 3/00, A61K 8/04, A61K 31/20, A61K 8/36

(54) **COMPOSITION LIPIDIQUE À BASE DE TRIACYLGLYCÉROL**
LIPIDZUSAMMENSETZUNG AUF TRIACYLGLYCEROLBASIS
TRIACYLGLYCEROL-BASED LIPID COMPOSITION

(30) Priorité: 21.06.2013 FR 1355963
(43) Date de publication de la demande: 18.05.2016
(73) Titulaire: Avril, 75008 Paris (FR); Universite Paris-Sud, 91405 Orsay (FR)
(72) Inventeur: THEVENIEAU, France, F-28000 Chartres (FR); SAMBOU, Sophie, F-95470 Fosses (FR); VIROLLE, Marie-Joëlle, F-91440 Bures Sur Yvette (FR); DULERMO, Thierry, F-78100 Saint Germain En Laye (FR)
(74) Mandataire: Monni, Richard
(86) Numéro de dépôt international: PCT/IB2014/062488
(87) Numéro de publication internationale: WO 2014/203219

(56) Documents cités:
- EP-A1- 2 390 341
- US-A1- 2005 143 463
- KIMOTO-NIRA H ET AL: "Bile resistance in Lactococcus lactis strains varies with cellular fatty acid composition: Analysis by using different growth media", INTERNATIONAL JOURNAL OF FOOD MICROBIOLOGY, ELSEVIER BV, NL, vol. 131, no. 2-3, 31 mai 2009 (2009-05-31), pages 183-188, XP026042779, ISSN: 0168-1605, DOI: 10.1016/J.IJFOODMICRO.2009.02.021 [extrait le 2009-03-03]

## Description

La présente invention concerne des compositions lipidiques à base de triacylglycérols (TAGs) et ses différentes applications.

Le lubrifiant est une substance grasse qui permet de réduire les frottements entre deux surfaces. Il est composé en majeure partie d'une huile de base et d'additifs qui lui confèrent des propriétés particulières. Les huiles utilisées sont généralement d'origine minérale, végétale, animale ou synthétique, et peuvent être recyclées.

Suite à l'appauvrissement des ressources pétrolières et à la parution de nombreuses études démontrant l'impact négatif sur l'environnement des composés issus de la pétrochimie, les huiles d'origine végétale ou animale tendent à se substituer aux huiles d'origine pétrochimique pour des applications dans de nombreux domaines, en particulier dans le domaine des lubrifiants.

L'utilisation de biolubrifiants a émergée au cours de ces dernières années (huile d'origine végétale ou animale) et bien qu'avantageuse d'un point de vue écologique, elle reste toujours marginale (moins de 5% du marché des lubrifiants) du fait des coûts de production très élevés par rapport aux lubrifiants d'origine pétrochimique et aux difficultés d'approvisionnement en huiles végétales nécessitant des cultures de grandes envergures (cultures oléagineuses) souvent destinées à l'alimentation humaine ou animale.

Pourtant, l'utilisation de ces biolubrifiants s'avère très avantageuse, notamment dans les applications où la récupération des lubrifiants est quasi-impossible ou sujette à des pertes accidentelles (dans les sols et les eaux), car leur impact sur l'environnement est limité et ils permettent de minimiser les risques sanitaires.

C'est pourquoi, dans une logique de développement de produits plus respectueux de l'environnement, des huiles végétales et animales se trouvent utilisées dans diverses applications.

Le document « Acides gras : nomenclature et sources alimentaires », de Cuvier C Cabaraux J.-F., Dufrasne I., Hornick J.-L., Istasse L. Manuscrit Ann. Méd. Vét., 2004, 148, 133-140*.,* décrit le contenu en acides gras des compositions lipidiques de différents produits d'origine animale ou végétale : huile de colza, de maïs, tournesol, d'olive, de lin etc.

Des compositions lipidiques à base de triacylglycérols sont décrites dans la demande de brevet NESTE OIL OYJ EP 2390341, issues d'un procédé de synthèse de lipides par un micro-organisme. L'étude porte sur l'accumulation dans le cytoplasme de TAGs par le microorganisme.

Il existe un besoin en une source lipidique alternative biodégradable, non toxique et/ou biosourcée.

A cet effet, l'invention concerne une composition lipidique comprenant des acides gras majoritairement sous forme de triacylglycérols, dans laquelle :
- au moins 50% des acides gras sont des acides 14-méthyl pentadécanoïque, notamment au moins 55% sont des acides gras sont des acides 14-méthyl pentadécanoïque, et
- au moins 8% des sont acides gras sont des acides 12-méthyl tétradécanoïque,
les pourcentages étant exprimés en poids des acides gras totaux de la composition.

Par « composition lipidique», on entend une composition comprenant au moins 50% de lipides, pourcentage exprimé en poids total de la composition. La composition comprend avantageusement au moins 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% ou 99% de lipides, pourcentages exprimés en poids total de la composition. La composition peut comprendre tout autre composant en respect avec les proportions en lipide précitées.

Cette composition lipidique comprend des acides gras qui sont majoritairement sous forme de TAGs.

Par « acides gras majoritairement sous forme de triacylglycérols», on entend qu'au moins la moitié (au moins 50% en poids total des acides gras totaux de la composition) des acides gras présents dans la composition est estérifiée sur une molécule de triacylglycérol. La composition comprend avantageusement au moins 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% ou 99% des acides gras sous forme de triacylglycérols, ces pourcentages sont exprimés en poids total des acides gras de la composition.

La composition selon l'invention constitue donc une source intéressante et alternative en TAGs, facilement extractibles et présents en grande quantité. Les TAGs, étant les principaux composants des huiles, ce sont des composés chimiques à fort potentiel industriel. Ils sont en particulier connus pour avoir un pouvoir lubrifiant naturel et une bonne adhésivité aux surfaces métalliques.

De plus, comme il sera précisé ci-après, la composition selon l'invention comprend des acides gras à chaîne aliphatique courte pouvant comprendre des motifs particuliers, comme par exemple, la présence d'un groupement méthyle sur l'avant dernier ou l'avant avant dernier atome de carbone de la chaîne, ou encore d'un groupement hydroxyle.

Avantageusement, la composition selon l'invention comprend une majorité d'acides gras saturés. Elle comprend également des acides gras insaturés en moindre quantité, lesdits les acides gras insaturés présentant un nombre d'insaturations réduit (de préférence moins de 3). Ces différentes caractéristiques confèrent à la composition des propriétés améliorées : un meilleur comportement de l'huile à basse température, une résistance à la thermo-oxydation et/ou une meilleure fluidité.

Par ailleurs, une composition selon l'invention peut être obtenue par synthèse chimique et/ou par synthèse biologique par un procédé mettant en oeuvre un microorganisme.

Les acides gras de la composition selon l'invention étant disponibles à la vente, l'homme du métier peut facilement réaliser une réaction d'estérification en présence de glycérol afin d'obtenir un mélange de monoacylglycérols, diacylglycérols et triacylglycérols. La nature des acides gras et les proportions selon l'invention seront prédéterminées, et prédéterminables aisément par l'homme du métier.

La composition peut également être synthétisée par un microorganisme, en milieu nutritionnel comprenant une source de carbone, notamment du glycérol, du glucose ou toute autre source de carbone moins raffinée provenant par exemple des déchets de l'agro-industrie tels que des déchets industriels organiques ou des restes, des déchets organiques agricoles, des déchets urbains ou des déchets microbiens, ou des mélanges de ceux-ci.

L'augmentation de l'accumulation en TAGs dans le cytoplasme du microorganisme peut être favorisée par l'utilisation du glycérol comme source principale de carbone, par une étape d'hypercompensation en phosphate ou azote, ou par un mélange (Glycérol et hypercompensation).

La culture dans un milieu riche puis dans un milieu appauvri en nutriment est connue de l'état de la technique comme l' « hypercompensation ».

Dans le cadre de l'hypercompensation en phosphate organique ou minéral, il est avantageusement ajouté au milieu de pré-culture de 2,5 mM à 50 mM de phosphate inorganique, préférentiellement de 3 mM à 40 mM, plus particulièrement de 4 mM à 30 mM, encore plus particulièrement de 5 mM à 20mM, notamment de 5 mM à 10 mM de phosphate inorganique.

Avantageusement, le glycérol qui est facilement accessible à partir de la filière oléagineuse, peut être utilisé comme source de carbone brut ou raffiné.

L'acide 14-méthyl pentadécanoïque est également appelé par son nom usuel « acide gras iso-hexadécanoïque » ou par sa nomenclature physiologique « iso C16 :0 ».

L'acide 12-méthyl tétradécanoïque, de son nom usuel « acide gras antéiso-heptadécanoïque», est également nommé par sa nomenclature physiologique « antéiso C15 :0 ».

De préférence, la composition comprend au moins 55% de triacylglycérols, le pourcentage est exprimé en poids total de la composition. De façon encore avantageux, la composition comprend au moins 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% ou 99% de triacylglycérols, les pourcentages étant exprimés en poids total de la composition lipidique.

Avantageusement, la composition comprend majoritairement des composés issus de la biomasse (bio-synthétisés), de façon encore avantageuse, tous les composés de la composition sont issus de la biomasse.

De façon avantageuse, la composition lipidique de l'invention comprend au moins 55 % d'acide 14-méthyl pentadécanoïque, en poids des acides gras totaux de la composition. De préférence, la composition comprend au moins 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% et 99% d'acide 14-méthyl pentadécanoïque, pourcentage exprimé en poids des acides gras totaux de la composition.

Selon un mode de réalisation avantageux, la composition selon l'invention définie ci-dessus comprend en outre de 6,5% à 20% d'acide gras 12-méthyl tridécanoïque, les pourcentages étant exprimés en poids des acides gras totaux de la composition.

L'acide 12-méthyl tridécanoïque est également appelé par son nom usuel « acide gras iso-tétradécanoïque » ou par sa nomenclature physiologique «iso C14:0».

De préférence, la composition selon l'invention comprend 7%, 8%, 9%, 10%, 11%, 12%, 13%, 14%, 15%, 16%, 17%, 18%, 19% ou 20% d'acide gras 12-méthyl tridécanoïque, les pourcentages étant exprimés en poids total de la composition.

De façon également avantageuse, la composition selon l'invention comprend au moins 50% d'acide 14-méthyl pentadécanoïque, au moins 8% d'acides 12-méthyl tétradécanoïque et de 6,5% à 20% d'acide gras 12-méthyl tridécanoïque, les pourcentages étant exprimés en poids des acides gras totaux de la composition.

Selon un mode de réalisation avantageux, la composition selon l'invention définie ci-dessus comprend en outre de 6,5% à 20% d'acide gras 14-méthyl hexadécanoïque, les pourcentages étant exprimés en poids des acides gras totaux de la composition.

L'acide 14-méthyl hexadécanoïque est également appelé par son nom usuel « acide gras antéiso-heptadécanoïque » ou par sa nomenclature physiologique «antéiso C17:0».

De préférence, la composition selon l'invention comprend 7%, 8%, 9%, 10%, 11%, 12%, 13%, 14%, 15%, 16%, 17%, 18%, 19% ou 20% d'acide gras 14-méthyl hexadécanoïque, les pourcentages étant exprimés en poids total de la composition.

De façon également avantageuse, la composition comprend au moins 50% d'acide 14-méthyl pentadécanoïque, au moins 8% d'acides 12-méthyl tétradécanoïque, de 6,5% à 20 % d'acide gras 12-méthyl tridécanoïque et/ou de 6,5% à 20 % d'acide gras 14-méthyl hexadécanoïque, les pourcentages étant exprimés en poids des acides gras totaux de la composition.

Selon un mode de réalisation avantageux, la composition selon l'invention définie ci-dessus comprend au moins 8 % d'acide gras 12-méthyl tétradécanoïque, le pourcentage étant exprimé en poids des acides gras totaux de la composition.

Selon un mode de réalisation avantageux, la composition selon l'invention définie ci-dessus comprend des acides gras ayant une chaîne carbonée de 8 à 22 atomes de carbone.

De préférence, la chaîne carbonée a 9, 10, 11, 12, 13, 14, 15, 16, 17, 18, 19 ou 20 atomes de carbone, de façon encore préférée de 14 à 17 atomes de carbone.

Les acides gras, linéaires ou ramifiés, peuvent être hydroxylés et/ou méthylés, i.e. les chaînes carbonées sont substituées par au moins un groupement hydroxyle et/ou méthyle.

La position du groupement méthyle varie avantageusement entre l'avant dernier atome de carbone en partant du groupement méthyle terminal (iso) et l'antépénultième (antéiso).

Avantageusement, la composition selon l'invention comprend majoritairement des acides gras saturés. De préférence, la composition comprend au moins 60% d'acides gras saturés, pourcentage exprimé en poids total des acides gras de la composition.

Avantageusement, au moins 50% des acides gras insaturés présents dans la composition selon l'invention sont mono insaturés, pourcentage exprimé en poids total des acides gras insaturés. De préférence, au moins 70% des acides gras insaturés sont mono-insaturés, de façon encore préférée 90% des acides gras insaturés sont mono-insaturés.

Selon un mode de réalisation encore plus avantageux, la composition selon l'invention comprend au moins les acides gras choisis parmi l'acide 12-méthyl tridécanoïque (iso C14:0/ acide iso tétradécanoïque), l'acide tétradécénoïque (C14:1), l'acide 12-méthyl tétradécanoïque (antéiso C15:0/ acide antéiso pentadécanoïque), l'acide pentadécanoïque (C15:0), l'acide 14-méthyl pentadécanoïque (isoC16:0/ acide iso hexadécanoïque), l'acide 14-méthyl pentadécénoïque (iso C16:1/ acide iso hexadécénoïque), l'acide hexadécanoïque (C16:0), l'acide hexadécénoïque (C16:1), l'acide 15-méthyl hexadécanoïque (iso C17:0/ acide iso heptadécanoïque), l'acide heptadécanoïque (C17:0), l'acide heptadécénoïque C17:1 et/ou l'acide 14-méthyl hexadécanoïque (antéiso C17:0/ acide antéiso heptadécanoïque).

De préférence, tous les acides gras de la composition sont choisis parmi ceux cités précédemment.

Selon un mode de réalisation encore plus avantageux, les acides gras totaux de la composition sont présents dans les proportions telles que définies ci-après :
- de 6,7% à 7,7 % d'iso C14:0,
- de 3,1% à 4 % C14:1,
- de 9,8 à 11 % d'antéiso C15:0,
- de 0,1% à 0,7 % de C15:0,
- de 61,5% à 62,5% d'isoC16:0,
- de 0,5% à 1,5%d'isoC16:1,
- de3,1%à4%d'C16:0,
- de 0,1% à 7,7 % d'C16:1,
- de 0,5% à 1,5% de C17:0,
- de 0,5% à 1,5 % de C17:1,
- de 1,1% à 2,1 % d'isoC17:0, et
- de 6,7% à 7,7 % d'anteisoC17:0,
les pourcentages étant exprimés en poids des acides gras totaux de la composition.

La composition selon l'invention peut comprendre, en outre, tout autre type de lipides et/ou des impuretés, les lipides étant par exemple des acides gras libres, des oligomères, des stérols, des esters d'acide gras, des esters de stérol, des monoacylglycérols et/ou des diacylglycérols.

La composition peut comprendre jusqu'à 49 % d'impuretés, pourcentage exprimé en poids total de la composition. Les impuretés peuvent être des débris cellulaire tels que les protéines, les lipides membranaires tels que les sphingolipides, phospholipides ou autres, ou des acides nucléiques etc. De préférence, la composition comprend 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47% ou 48% d'impureté, pourcentage exprimé en poids total de la composition.

Avantageusement, la proportion de débris a la composition suivante :
- de 35% à 45% de protéines,
- de 2% à 10% d'ADN,
- de 10% à 20% d'ARN,
- de 10% à 15% de peptidoglycanes,
- de 2 à 5% d'hydrocarbures,
- de 5% à 10% d'acide téichoïque,
- de 0,1% à 5% de molécules solubles, et
- de 5% à 10% de cendres,
les pourcentages étant exprimés en poids total de la composition de débris cellulaires.

Elle peut comprendre en outre des molécules solubles telles que les antibiotiques, les sidérophores et les vitamines.

Selon encore un autre aspect avantageux, l'invention concerne également un extrait de microorganisme comprenant une composition lipidique telle que décrit ci-avant.

De façon avantageuse, la composition lipidique représente au moins 10% en poids de l'extrait total, de préférence au moins 40%. De façon encore plus avantageuse, la composition lipidique représente 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% ou 99% en poids de l'extrait total.

L'extrait de microorganisme susmentionné comprend en outre des débris cellulaires spécifiques du microorganisme en question. Les constituants majeurs (atomes principaux) de la biomasse cellulaire de plusieurs bactéries sont donnés à titre d'exemple dans le tableau 1 ci-dessous :

**Tableau 1 : Constituants majeurs de la biomasse cellulaire**

| Microorganismes | Composition de la biomasse |
|---|---|
| *Escherichia coli* | C H 1,77 O 0,49 N 0,24 |
| *Klebsiella aerogenes* | C H 1,75 O 0,43 N 0,22 |
| *Paraccocus denitrificans* | C H 1,81 O 0,51 N 0,20 |
| *Pseudomonas C12B* | C H 2,00 O 0,52 N 0,23 |
| *Aerobacter aerogenes* | C H 1,83 O 0,55 N 0,25 |
| *Streptomyces cattleya* | C H 1,60 O 0,58 N 0,17 |
| *Streptomyces clavuligerus* | C H 1,82 O 0,62 N 0,21 |
| *Corynebacterium glutamicum* | C H 1,77 O 0,49 N 0,22 |
| *Brevibacterium ssp* | C H 1,62 O 0,50 N 0,12 |
| Moyenne | C H 1,80 O 0,50 N 0,20 |

Ces résultats ont été obtenus par analyse avec un appareil Thermo Fisher Flash 2000 série CHNS/O, commercialisé par Thermo Scientific.

De préférence, l'extrait de micro-organisme selon l'invention comprend les constituants cellulaires classiques : des protéines, de l'acide désoxyribonucléique (ADN), de l'acide ribonucléique (ARN), des phospholipides, des triacylglycérols, des molécules solubles, des peptidoglycanes, de l'acide téichoïque, des carbohydrates et des cendres cellulaires.

Selon un aspect de l'invention, la présente invention concerne également l'utilisation d'une composition lipidique telle que décrit ci-avant ou d'un extrait tel que décrit ci-avant, pour la préparation de biocarburant, de lubrifiant, de tensio-actifs, de revêtements (peintures, encres,..), de solvants, d'ingrédients alimentaires ou comme intermédiaires de synthèse pour l'oléochimie.

Il est possible d'obtenir du biodiesel par des procédés connus, par exemple un procédé de transestérification des triglycérides présents dans la composition selon l'invention ou l'extrait de microorganisme selon l'invention, pour la production d'esters méthyliques d'huiles qui servent de carburants sous la dénomination de biodiesel.

La transestérification est une réaction comportant trois étapes réversibles en série dans lesquelles les triglycérides sont convertis en diglycérides, puis ceux-ci sont convertis en monoglycérides et enfin les monoglycérides sont convertis en esters (biodiesel) et glycérol (co-produit).

Au cours de la réaction de transestérification, les huiles ou graisses réagissent avec un alcool à chaine courte (généralement le méthanol) en présence d'un catalyseur.

Cette réaction peut être effectuée par catalyse homogène, avec des catalyseurs solubles dans le milieu réactionnel, ou par catalyse hétérogène, avec des catalyseurs totalement insolubles dans le réactif.

A l'heure actuelle, la catalyse homogène est la technique la plus généralement utilisée dans les procédés de production de biodiesel. La transestérification peut être réalisée par catalyse basique ou acide. Une plus grande réactivité est généralement obtenue en milieu basique.

Trois grandes classes de catalyseurs existent : les catalyseurs basiques, les catalyseurs acides et les autres catalyseurs.

On peut citer à titre d'exemple, les hydroxydes, alcoolates ou savons de métaux alcalins ou alcalino-terreux (Li, Na, K, Ca, Ba, Cs...), les aminés de la famille des guanidines ; les acides minéraux (HCl, H2SO4), les acides sulfoniques, les résines échangeuses d'ions (acide fort), les zéolithes, les alcoolates de titane (Ti (OBu) 4, Ti (OiPr)4), les oxydes de divers métaux (Sn, Mg, Zn, Ti, Pb).

Le biodiesel obtenu peut être utilisé en mélange avec du diesel d'origine fossile ou pur comme carburant.

Le lubrifiant obtenu à partir de la composition selon l'invention peut comprendre en outre des additifs généralement utilisés dans une composition de lubrifiant.

L'invention concerne également une composition pharmaceutique ou cosmétique comprenant une composition lipidique telle que décrite ci-avant, ou un extrait tel que défini ci-avant, en association avec un véhicule pharmaceutiquement ou cosmétiquement acceptable.

Les acides carboxyliques, ou acides gras, susmentionnés de la composition sont capables de protéger avantageusement de la chaleur les fibres contenant de la kératine, et en particulier les acides gras de forme antéiso. La composition est donc adaptée pour son utilisation dans le coiffage temporaire à chaud de cheveux, notamment des cheveux humains.

L'invention concerne avantageusement une composition cosmétique comprenant une composition telle définie ci-dessus, en association avec une substance active raffermissante, ladite composition cosmétique étant notamment sous forme de mousse.

Une telle composition cosmétique peut être utilisée pour le coiffage des fibres kératiniques. Aussi, l'invention concerne avantageusement l'utilisation de la composition cosmétique susmentionnée pour le coiffage des fibres kératiniques.

La composition selon l'invention peut être transformée en ammonium quaternaire et être ainsi utilisée (à hauteur de 10%) dans des produits cosmétiques comprenant au moins une substance active raffermissante et qui se présentent en particulier sous forme de mousse. Il est ainsi possible d'obtenir un résultat de coiffage très durable, notamment avec une humidité de l'air importante.

Les sels d'ammonium quaternaire ou aminés quaternaires sont produits par une réaction de quaternisation. Il s'agit généralement d'une réaction entre une alkyle amine (ou alkyl amidoamine) et un halogénure d'alkyle ou sel d'alkyle. Par exemple, la réaction est réalisée en deux étapes, une première étape au cours de laquelle les acides gras de la composition sont mis à réagir avec un excès de diméthylaminopropylamine pour former un alkylamidoamine, et une seconde étape lors de laquelle l'alkylamidoamine de la première étape réagit à son tour avec du diéthyl sulfate en présence de butylène glycol pour former l'ammonium quaternaire final qui sera utilisé dans la formulation cosmétique d'intérêt.

Une substance active raffermissante est connue de l'homme du métier et peut être notamment une cire végétale ou animale, par exemple la cire d'abeille.

La composition comprend avantageusement des dérivés esters choisis parmi le groupe constitué par les esters de stérols, les esters de sucres, les esters de 2-éthylhexanol, les esters d'alcools supérieurs, seul ou en mélange de ceux-ci.

Ces dérivés peuvent entrer dans la formulation de produits cosmétiques ou de médicaments à usage externe, notamment pour des applications topiques cutanées.

L'association de la composition selon l'invention avec un véhicule pharmaceutiquement acceptable permet par exemple d'optimiser l'hydratation de la peau, le lissage des cheveux ou un effet élastique sur les tissus. Il peut par ailleurs avoir d'autres effets comme par exemple l'assimilation facilitée d'un médicament.

Par exemple, il est possible d'encapsuler des principes actifs dans des vésicules lipidiques ayant une composition telle qu'elles fusionnent aisément avec les lipides membranaires ce qui facilite grandement l'entrée du principe actif dans les cellules. Avantageusement, la composition lipidique ou l'extrait de microorganismes peuvent être utilisés en tant que médicament ou alicament (complément alimentaire).

Elle peut avoir une application notamment dans le traitement de problèmes de peau ou également en tant que cicatrisant.

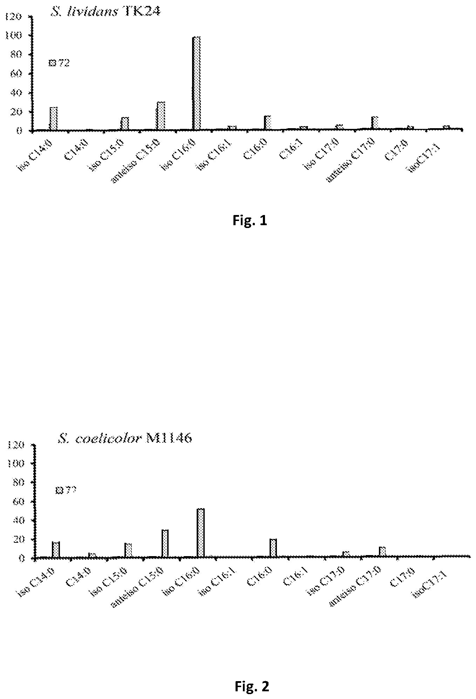

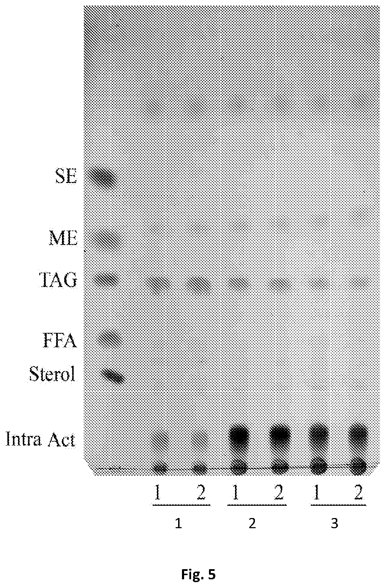

L'invention sera mieux comprise à la lecture des figures et des exemples qui sont donnés uniquement à titre illustratif et ne présentent aucun caractère limitatif.
**La** **figure 1** représente le profil lipidique de compositions lipidiques obtenues par culture de la souche *Streptomyces lividans* TK24 en milieu comprenant du glucose comme source principale de carbone (55mM) pendant 72 heures ou 96 heures. Les différents acides gras sont donnés en abscisse et la quantité en µg/mg de biomasse sèche est exprimée en ordonnée.
**La** **figure 2** représente le profil lipidique de compositions lipidiques obtenues par culture de la souche *Streptomyces coelicolor* M1146 en milieu comprenant du glucose comme source principale de carbone (55mM) pendant 72 heures ou 96 heures. Les différents acides gras sont donnés en abscisse et la quantité en µg/mg de biomasse sèche est exprimée en ordonnée.

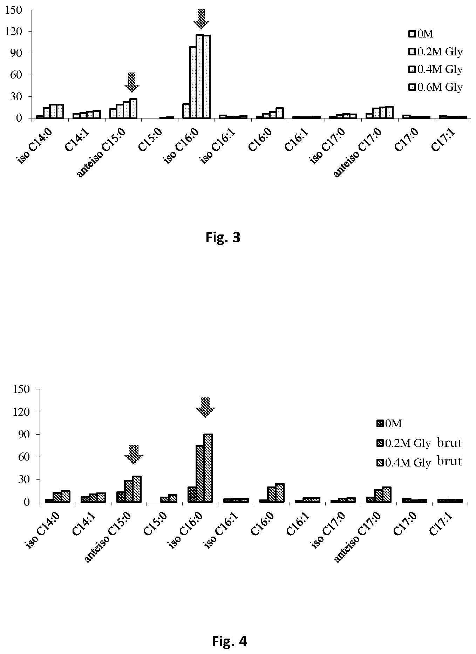

La **figure 3** représente le profil lipidique de compositions lipidiques obtenues par culture de la souche *Streptomyces lividans* TK24 en présence de glycérol purifié comme source principale de carbone, pendant 96 heures, à différentes concentrations. Les différents acides gras présents sont donnés en abscisse et la quantité en µg/mg de biomasse sèche est exprimée en ordonnée.
**La** **figure 4** représente le profil lipidique de compositions lipidiques obtenues par culture de la souche *Streptomyces lividans* TK24 en présence de glycérol non raffiné, c'est-à-dire brut, comme source principale de carbone, pendant 96 heures, à différentes concentrations.
**La** **figure 5** représente une chromatographie sur couche mince, de compositions lipidiques selon l'invention. Ces compositions sont obtenues par deux cultures de trois souches différentes de *streptomyces (1 : Streptomyces lidivans TK24 sauvage ; 2: Streptomyces lidivans* TK24 mutante pour le gène *ppk; 3: Streptomyces coelicolor M145).*

Les différents acides gras présents sont donnés en abscisse et la quantité en µg/mg de biomasse sèche est exprimée en ordonnée.

### Exemple 1 : Synthèse d'une composition lipidique selon l'invention, par un procédé de culture d'une souche de streptomyces.

Certaines espèces de *streptomyces,* comme de nombreux autres micro-organismes, ont la capacité naturelle à accumuler dans leur cytoplasme plus de 40% de leur poids sec en TAGs.

Les *streptomyces,* comme de nombreux autres micro-organismes, ont la capacité d'accumuler dans leur cytoplasme en moyenne 20% de leur poids en TAGs.

Les TAGs sont constitués d'une molécule de glycérol et de trois acides gras (de nature identique ou différente) estérifiés sur les groupements hydroxyle du glycérol.

Dans cet exemple, la production de la composition lipidique selon l'invention a été réalisée à partir d'une culture de *streptomyces*, mais aurait aussi bien pu être réalisée à partir d'autres souches bactriennes de différents genres de l'ordre des actinomycètes tels que *Rhodococcus, Micrococcus* ou *Actinomyces.*

### A. Différents procédés de culture

Plusieurs souches de Streptomyces sont mises en culture suivant différents procédés détaillés ci-dessous : culture avec du glucose comme source principale de carbone (a), culture avec du glycérol comme source principale de carbone (b), et culture glycérol combinée à une hypercompensation en phosphate (c).

On entend par « source principale de carbone » une quantité de composé carboné métabolisable par les bactéries et permettant de produire de l'énergie. La source majoritaire de carbone représente plus de 90% de l'apport en carbone dans le milieu de culture ou de pré-culture.

### (a) Culture de Streptomyces lividans TK24 et Streptomyces coelicolor M1146 en milieu enrichi en glucose

Des spores (10⁶) de deux souches de *Streptomyces lividans* TK24 et *Streptomyces coelicolor* M1146 ont été étalées sur un milieu gélosé R2YE (Glucose 55mM, 1mM-Pi) sur la surface duquel a été déposé un cellophane poreux aux éléments nutritifs du milieu. Ces souches ont été mises à pousser pendant 72h à 28°C.

Le milieu R2YE a la composition suivante :
- K₂SO₄ (1,4 .10⁻³ mol/L et 0,25 g/L) MM=174 g/mol
- MgCl₂ 6H₂O (5.10⁻² mol/L et 10,12 g/L) MM=203 g/mol

### - Glucose ou glycérol

- Acides aminés de caséine (0.1 g/L)

Complémentation après autoclave en solide
- CaCl₂ 2H₂O (2.10⁻² mol/L et 2.94 g/L) MM=147 g/mol
- L-proline (2,6.10⁻² mol/L et 3 g/L) MM=115 g/mol
- TES buffer (2,5.10⁻² mol/L et 5.73 g/L) MM=2304 g/mol
- Oligoélément (2 mL/L)
- Yeast Extract (5 g/L)
- NaOH (5.10⁻³ mol/L et 0.2 g/L) MM=30 g/mol

La composition obtenue est analysée à la suite d'une étape d'extraction et selon les méthodes d'analyse décrites au point B. Les résultats sont présentés en figure 1 pour la culture de *Streptomyces lividans* TK24 et en figure 2 pour la culture de *Streptomyces coelicolor* M1146.

On observe en figure 1, une composition présentant une quantité importante d'acide gras isoC16 :0 et antéisoC15 :0. Ces proportions sont celles de l'invention, c'est-à-dire, au moins 50% d'isoC16 :0 et au moins 8% d'antéisoC15 :0.

De façon identique, on observe en figure 2 une composition présentant une quantité importante d'acide gras isoC16 :0 et antéisoC15:0, les proportions étant également celles de l'invention, c'est-à-dire, au moins 50% d'isoC16 :0 et au moins 8% d'antéisoC15 :0.

### (b) Culture de Streptomyces lividans TK24 en milieu enrichi en glycérol

Des spores (10⁶) de deux souches de *Streptomyces lividans* TK24 ont été étalées sur un milieu gélosé R2YE contenant du glycérol purifié ou brut (Glycérine Prolea) à différentes concentrations, sur la surface duquel a été déposé un cellophane poreux aux éléments nutritifs du milieu. Ces souches ont été mises à pousser pendant 96h à 28°C.

La composition obtenue est analysée à la suite d'une étape d'extraction et selon les méthodes d'analyse décrites au point B. Les résultats sont présentés en figure 3 pour la culture sur glycérol purifié et en figure 4 pour la culture en glycérol non raffiné.

Le glycérol brut peut être purifié par traitement au charbon actif pour éliminer les impuretés organiques par traitement alcalin pour éliminer les esters du glycérol qui n'ont pas réagi, et par l'échange d'ions pour éliminer les sels. Le glycérol obtenu par une série de distillation présente une de pureté élevée (> 99,5%), on dit alors qu'il est raffiné.

On observe en figure 3, qu'en présence d'une quantité de glycérol raffiné supérieure à 0,2M, la composition comprend une quantité en acide gras isoC16 :0 environ de 100µm/mg du poids sec et environ 30 µm/mg du poids sec d'acide gras antéisoC15 :0.

De façon équivalente, on observe en figure 4 qu'en présence de quantité en glycérol non raffiné supérieure à 0,2M, la composition comprend une quantité en acide gras isoC16 :0 d'environ de 80µm/mg du poids sec et environ 30 µm/mg du poids sec d'acide gras antéisoC15 :0.

### (c) Culture de Streptomyces coelicolor M145 en milieu enrichi en glycérol combiné à une hyper-compensation phosphate (P)

Une souche de *Streptomyces lividans* TK24, a été cultivée pendant 96h en milieu liquide R2YE contenant du glycérol (0,2M) et phosphate (1mM).

La composition obtenue en acides gras, suite aux étapes d'extraction et d'analyse décrites au point B., est présentée dans le tableau 2 suivant :

**Tableau 2 : Composition des acides gras présents dans la composition**

| **Acides gras** | **Quantité (g)¹** |
|---|---|
| iso C14:0 | 3,5 |
| C14:1 | 1,75 |
| **antéisoC15:0** | **5** |
| C15:0 | 0,1 |
| **isoC16:0** | **30** |
| isoC16:1 | 0,5 |
| C16:0 | 1,75 |
| C16:1 | 0,25 |
| isoC17:0 | 0,8 |
| anteisoC17:0 | 3,5 |
| C17:0 | 0,5 |
| C17:1 | 0,5 |

| | |
|---|---|
| ¹ : valeurs exprimées pour 100g du poids de la biomasse sèche. | |

On observe la composition comprend une quantité en acide gras isoC16:0 environ de 30g du poids sec et environ 5g du poids sec d'acide gras antéisoC15 :0. Ces proportions sont celles de l'invention, c'est-à-dire, au moins 50% d'isoC16 :0 et au moins 8% d'antéisoC15 :0.

La composition lipidique comprend des acides gras à chaîne courte (de C14 à C17) et en majorité saturés. La composition comprend aussi des acides gras méthylés (iso, antéiso) à plus de 40g du poids de la biomasse sèche.

On aurait obtenu des résultats semblables, si on avait procédé à la culture de *Streptomyces* en excès puis en limitation en azote (N) au lieu du phosphate, pour l'étape d'hyper compensation.

### B. Etape d'extraction de la composition grasse des cellules et détermination de la nature en acide gras de la composition (nature et quantité de chaque AG) :

La lyse cellulaire peut être réalisée par toute technique connue de l'état de l'art : par action d'un agent physique, chimique et/ou biologique. Dans l'invention, la lyse de *Streptomyces* et l'extraction des lipides a été faite selon la méthode décrite dans : « FOLCH, J., LEES, M. & SLOANE STANLEY, G. H. A simple method for the isolation and purification of total lipides from animal tissues. J. Biol. Chem. 226, 497-509 (1957) ».

Ainsi les lipides présents dans la composition sont caractérisés et quantifiés.

I a été utilisé un mélange chloroforme méthanol 2 :1 et des plaques de silice de type « silice G60, de dimension 20/20 cm et de 0,25 mm d'épaisseur » commercialisées par Merck (Allemagne) sont utilisées.

La séparation des lipides est effectuée par un système à deux solvants.

Le solvant A (migration à mi-plaque) se compose d'éthyl éther de pétrole/ d'éther/ d'acide acétique à des proportions telles que 20/20/0,8 (vol/vol/vol).

Le solvant B (migration complète) se compose de di-éthyl éther de pétrole/ d'éther à des proportions telles que 49/1 (vol/vol).

A la fin de la migration, la révélation est effectuée au MnCl₂ par la méthode de carbonisation : la plaque de silice est incubée pendant 1 minute dans une solution contenant 120 ml de méthanol, 120 ml d'eau, 0,8 g de MnCl2 et de 8 ml d'acide sulfurique et est ensuite chauffée dans une étuve à 100°C jusqu'à apparition des taches sombres des lipides.

L'identification des lipides est déterminée à partir des normes de migration lipidiques (Sigma-Aldrich).

La coloration des lipides a été ensuite enregistrée en utilisant le système d'imagerie LAS-3000 et le logiciel Multigauge de Fujifilm.

Les résultats obtenus sont présentés sur la figure X qui représente une chromatographie en couche mince. On constate que la composition comprend plus de 50% de TAG.

La composition en acide gras peut également être analysée en chromatographie en phase gazeuse (GC), après une étape de conversion en esters méthyliques nécessaire à l'analyse connu de l'homme du métier, selon la procédure *Browse et al. (1986).*

L'analyse GC d'esters méthyliques d'acide gras est réalisée avec un chromatographe 7890A (Agilent) à colonne capillaire d'un facteur quatre VF-23ms 30m * 0,25 mm.

Le gaz vecteur est l'hélium ayant une pression d'entrée de 1 mL/min.

Le programme de la température de la colonne est tel que définit ci-apres :
- débuter à 40°C pendant 1 minute,
- élever la température de la colonne jusqu' à 130°C par augmentations de 40°C par minute,
- maintenir 1 minute à une température de 130°C,
- élever la température de la colonne jusqu' à 160°C par augmentations d'1 °C par minute,
- maintenir 1 minute à 160 ° C,
- élever la température de la colonne jusqu' à 210°C par augmentations d'40°C par minute, et
- maintenir 4 minutes à 210 ° C.

Une fois le passage sur colonne terminé, l'identification des pics obtenue se fonde sur des comparaisons avec des temps de rétention normalisés (Sigma-Aldrich).

La quantification est faite par détection par ionisation de flamme à 270 ° C.

La quantité totale des acides gras est calculée à partir du ratio entre la somme des aires des pics FAME et l'aire du pic de l'ester méthylique de l'acide octodécanoïque (Sigma-Aldrich).

### Exemple 2 : Composition d'un extrait de S. coelicolor M145 comprenant la composition selon l'invention.

La composition de l'extrait de S. *coelicolor* M145 obtenu au point (c) est analysée ci-après.

La composition en acides aminés, en acides gras, en acides nucléiques et en sucres de S. *coelicolor* est basée sur celle publiée par Borodina (Borodina et al., 2005b).
- Atomes présents dans l'extrait de *Streptomyces coelicolor:* C H 1,62 O 0,45 N 0,25
- Nature des constituants :

**Tableau 3 : Composition de la biomasse de S. coelicolor M145 (µmol (gramme de cellules déshydratées)⁻¹)**

| Macromolécules | Données de la littérature (% CDW) | Poids calculé à partir de la composition théorique (%CDW) | RSD (%) |
|---|---|---|---|
| Protéines | 41,2 | 41,1 | 0,17 |
| ADN | 3,6 | 6,0 | 35,36 |
| ARN | 16,7 | 16,7 | 0,00 |
| Phospholipides | 2,7 | 3,3 | 14,14 |
| Triacylglycérols | 1,8 | 2,0 | 7,44 |
| Molécules solubles | 3,0 | 2,6 | 10,10 |
| Peptidoglycane | 11,0 | 11,4 | 2,53 |
| Carbohydrates | 4,4 | 4,4 | 0,00 |
| Acide téichoïque | 6,6 | 6,8 | 2,11 |
| Cendres | 9,0 | 5,7 | 31,75 |
| Total | 100,0 | 100,0 | 0,00 |

Les valeurs des principaux polymères entrant dans la composition de la biomasse de S. *coelicolor*, à savoir les protéines représentent 41,2% et les acides nucléiques 20,3% du poids sec.

Contrairement à la souche *coelicolor* M145, la souche de S. *coelicolor M*1146 (souche délétée pour les principales voies de biosynthèse d'antibiotiques), a un taux de TAGs qui atteint 30% en glucose et 40% en glycérol.

### Exemple 3: Composition selon l'invention utilisée comme huile de base dans un lubrifiant.

La composition selon l'invention peut être transestérifiée par du TMP (Triméthylol Propane) et utilisée comme huile de base dans un lubrifiant.

Dans cette utilisation, la part des composés saturés est majoritaire par rapport à celle des composés insaturés. En proportion relative, le pourcentage des composés insaturés est de l'ordre de 1 à 12% par rapport au poids de la composition.

Une telle composition présente l'avantage d'être plus stable à froid et à l'oxydation qu'un dérivé contenant majoritairement des acides gras insaturés. Par ailleurs la dite composition a pour avantage d'être plus accessible en terme de coût et de disponibilité par rapport aux dérivés d'acide isostéarique utilisés actuellement.

On obtient les propriétés de stabilité à froid et de stabilité d'oxydation mesurées généralement pour une composition lubrifiante à base d'huile de base type dérivé du TMP telles qu'indiquées ci-après :

| Propriétés | Norme | Unité | TMPTO (base acides Gras insaturés) = référence | TMPTI (base acide branchés saturés) |
|---|---|---|---|---|
| Viscosité (40°C) | ASTM D445 | mm²/s | 46 | 68 |
| Pour Point d'écoulement | ASTM D97 | °C | -50 | -40 |
| RPVOT* | ASTM D2272 | Minutes | 180 | 800 |

| | | | | |
|---|---|---|---|---|
| * RPVOT ou test d'oxydation de récipient sous pression en rotation, est réalisé sur la composition, plus 1,5% d'Additin RC 9321 (RheinChemie). | | | | |

L'utilisation d'huile comprenant la composition selon l'invention apporte ainsi un bon compromis entre la stabilité à froid et la stabilité à l'oxydation.

Par exemple, 5 à 95% d'huile avec ladite composition est incorporée dans une huile de base pour lubrifiant permet d'apporter ces propriétés.

## Revendications

1. Composition lipidique comprenant des acides gras majoritairement sous forme de triacylglycérols, dans laquelle :
- au moins 50% des acides gras sont des acides 14-méthyl pentadécanoïque, et
- au moins 8% des acides gras sont des acides 12-méthyl tétradécanoïque,
les pourcentages étant exprimés en poids des acides gras totaux de la composition.

2. Composition selon la revendication 1, dans laquelle au moins 55 % en poids des acides gras totaux de la composition sont des acides 14-méthyl pentadécanoïque.

3. Composition selon la revendication 1 ou 2, qui comprend en outre de 6,5% à 20 % d'acide gras 12-méthyl tridécanoïque, le pourcentage étant exprimé en poids des acides gras totaux de la composition.

4. Composition selon l'une quelconque des revendications 1 à 3, qui comprend en outre de 6,5% à 20 % d'acide gras 14-méthyl hexadécanoïque, le pourcentage étant exprimé en poids des acides gras totaux de la composition.

5. Composition selon l'une quelconque des revendications 1 à 4, dans laquelle les acides gras ont une chaîne carbonée de 8 à 22 atomes de carbone.

6. Composition selon l'une quelconque des revendications 1 à 5, qui comprend au moins les acides gras choisis parmi l'acide 12-méthyl tridécanoïque (iso C14:0/ acide iso tétradécanoïque), l'acide tétradécénoïque (C14:1), l'acide 12-méthyl tétradécanoïque (antéiso C15:0/ acide antéiso pentadécanoïque), l'acide pentadécanoïque (C15:0), l'acide 14-méthyl pentadécanoïque (isoC16:0/ acide iso hexadécanoïque), l'acide 14-méthyl pentadécénoïque (iso C16:1/ acide iso hexadécénoïque), l'acide hexadécanoïque (C16:0), l'acide hexadécénoïque (C16:1), l'acide 15-méthyl hexadécanoïque (iso C17:0/ acide iso heptadécanoïque), l'acide heptadécanoïque (C17:0), l'acide heptadécénoïque C17:1 et/ou l'acide 14-méthyl hexadécanoïque (antéiso C17:0/ acide antéiso heptadécanoïque).

7. Composition selon l'une quelconque des revendications 1 à 6, dans laquelle les acides gras totaux de la composition sont présents dans des proportions telles que définies ci-après :
- de 6,7% à 7,7 % d'iso C14:0,
- de3,1%à4%C14:1,
- de 9,8 à 11 % d'anteiso C15:0,
- de 0,1% à 0,7 % de C15:0,
- de 61,5% à 62,5% d'isoC16:0,
- de 0,5% à 1,5 % d'isoC16:1,
- de3,1%à4%d'C16:0,
- de 0,1% à 7,7 % d'C16:1,
- de 0,5% à 1,5 % de C17:0
- de 0,5% à 1,5 % de C17:1
- de 1,1% à 2,1 % d'isoC17:0
et
- de 6,7% à 7,7 % d'anteisoC17:0,
les pourcentages étant exprimés en poids des acides gras totaux de la composition.

8. Composition selon l'une quelconque des revendications 1 à 7, qui comprend en outre tout autre type de lipides et/ou des impuretés, les lipides étant des acides gras libres, des oligomères, des stérols, des esters d'acide gras, des esters de stérol, des monoacylglycérols et/ou des diacylglycérols.

9. Extrait de microorganisme comprenant une composition lipidique selon l'une quelconque des revendications 1 à 8.

10. Utilisation d'une composition selon l'une quelconque des revendications 1 à 8 ou d'un extrait selon la revendication 9, pour la préparation de biocarburant, de lubrifiant, de tensio-actifs, de revêtements, de solvants, d'ingrédients alimentaires ou comme intermédiaires de synthèse pour l'oléochimie.

11. Composition pharmaceutique ou cosmétique comprenant une composition lipidique selon l'une quelconque des revendications 1 à 8, ou d'un extrait selon la revendication 9, seul ou en association avec un véhicule pharmaceutiquement ou cosmétiquement acceptable.

12. Composition cosmétique selon la revendication 11, en association avec une substance active raffermissante, notamment sous forme de mousse.

13. Utilisation d'une composition cosmétique telle que définie dans la revendication 12, pour le coiffage des fibres kératiniques.

14. Composition lipidique selon l'une quelconque des revendications 1 à 8, ou extrait selon la revendication 9, pour son utilisation en tant que médicament.

## Patentansprüche

1. Lipidzusammensetzung, die Fettsäuren überwiegend in Form von Triacylglycerolen enthält, wobei
- mindestens 50 % der Fettsäuren 14-Methylpentadecansäuren sind und
- mindestens 8 % der Fettsäuren 12-Methyltetradecansäuren sind,
wobei die prozentualen Anteile in Gewicht der Gesamtfettsäuren der Zusammensetzung ausgedrückt sind.

2. Zusammensetzung nach Anspruch 1, wobei mindestens 55 Gew.- % der Gesamtfettsäuren der Zusammensetzung 14-Methyl-pentadecansäuren sind.

3. Zusammensetzung nach Anspruch 1 oder 2, die ferner 6,5 % bis 20 % 12-Methyl-tridecan-Fettsäure enthält, wobei der prozentuale Anteil in Gewicht der Gesamtfettsäuren der Zusammensetzung ausgedrückt ist.

4. Zusammensetzung nach einem der Ansprüche 1 bis 3, die ferner 6,5 % bis 20 % 14-Methyl-hexadecan-Fettsäure enthält, wobei der prozentuale Anteil in Gewicht der Gesamtfettsäuren der Zusammensetzung ausgedrückt ist.

5. Zusammensetzung nach einem der Ansprüche 1 bis 4, wobei die Fettsäuren eine Kohlenstoffkette mit 8 bis 22 Kohlenstoffatomen haben.

6. Zusammensetzung nach einem der Ansprüche 1 bis 5, die ferner mindestens die Fettsäuren enthält ausgewählt aus der 12-Methyl-tridecan-Säure (iso-C14:0/iso-Tetradecansäure), der Tetradecensäure (C14:1), der 12-Methyl-tetradecansäure (anteiso-C15:0/anteisopentadecansäure), der Pentadecansäure (C15:0), der 14-Methyl-pentadecansäure (isoC16:0/iso-Hexadecansäure), der 14-Methyl-pentadecensäure (C16:1), iso-Hexadecensäure), der Hexadecansäure (C16:0), der Hexadecensäure (C16:1), der 15-Methyl-hexadecansäure (iso-C17:0/iso-Heptadecansäure), der Heptadecansäure (C17:0), der Heptadecensäure (C17:1) und/oder der 14-Methyl-hexadecansäure (anteiso-C17:0/anteiso-heptadecansäure).

7. Zusammensetzung nach einem der Ansprüche 1 bis 6, wobei die Gesamtfettsäuren der Zusammensetzung in den nachstehend definierten Anteilen vorhanden sind:
- 6,7 % bis 7,7 % iso-C14:0,
- 3,1 % bis 4 % C14:1,
- 9,8 % bis 11 % anteiso-C15:0,
- 0,1 % bis 0,7 % C15:0,
- 61,5 % bis 62,5 % iso-C16:0,
- 0,5 % bis 1,5 % iso-C16:1,
- 3,1 % bis 4 % C16:0,
- 0,1 % bis 7,7 % C16:1,
- 0,5 % bis 1,5 % C17:0,
- 0,5 % bis 1,5 % C17:1,
- 1,1 % bis 2,1 % iso-C17:0
und
- 6,7 % bis 7,7 % anteiso-C17:0,
wobei die prozentualen Anteile in Gewicht der Gesamtfettsäuren der Zusammensetzung ausgedrückt sind.

8. Zusammensetzung nach einem der Ansprüche 1 bis 7, die ferner jeden beliebigen anderen Lipid-Typ und/oder Verunreinigungen enthält, wobei die Lipide freie Fettsäuren, Oligomere, Sterine, Fettsäureester, Sterinester, Monoalkylglycerole und/oder Diacylglycerine sind.

9. Mikroorganismenextrakt, enthaltend eine Lipidzusammensetzung nach einem der Ansprüche 1 bis 8.

10. Verwendung einer Zusammensetzung nach einem der Ansprüche 1 bis 8 oder eines Extrakts nach Anspruch 9, für die Herstellung von Biokraftstoff, von Schmierstoff, von Tensiden, von Beschichtungen, von Lösemitteln, von Lebensmittelzutaten oder als Synthesezwischenprodukt für die Oleochemie.

11. Pharmazeutische oder kosmetische Zusammensetzung, enthaltend eine Lipidzusammensetzung nach einem der Ansprüche 1 bis 8, oder einen Extrakt nach Anspruch 9, allein oder in Verbindung mit einem pharmazeutisch oder kosmetisch verträglichen Träger.

12. Kosmetische Zusammensetzung nach Anspruch 11, in Verbindung mit einem straffenden Wirkstoff, insbesondere in Form von Schaum.

13. Verwendung einer kosmetischen Zusammensetzung nach Anspruch 12, zum Formen der Keratinfasern.

14. Lipidzusammensetzung nach einem der Ansprüche 1 bis 8, oder Extrakt nach Anspruch 9, für ihre bzw. seine Verwendung als Arzneimittel.

## Claims

1. Lipid composition comprising fatty acids predominantly in the form of triacylglycerols, wherein:
- at least 50 % of the fatty acids are 14-methyl pentadecanoic acids, and
- at least 8 % of the fatty acids are 12-methyl tetradecanoic acids,
the percentages being expressed by weight of the total fatty acids of the composition.

2. Composition according to claim 1, wherein at least 55 % by weight of the total fatty acids of the composition are 14-methyl pentadecanoic acids.

3. Composition according to claim 1 or 2, which further comprises from 6.5 % to 20 % of 12-methyl tridecanoic fatty acids, the percentage being expressed by weight of the total fatty acids of the composition.

4. Composition according to any one of claims 1 to 3, which further comprises from 6.5 % to 20 % of 14-methyl hexadecanoic fatty acids, the percentage being expressed by weight of the total fatty acids of the composition.

5. Composition according to any one of claims 1 to 4, wherein the fatty acids have a carbon chain of 8 to 22 carbon atoms.

6. Composition according to any one of claims 1 to 5, which comprises at least the fatty acids selected from 12-methyl tridecanoic acid (iso C14:0/ iso tetradecanoic acid), tetradecenoic acid (C14:1), 12-methyl tetradecanoic acid (anteiso C15:0/ anteiso pentadecanoic acid), pentadecanoic acid (C15:0), 14-methyl pentadecanoic acid (iso C16:0/ iso hexadecanoic acid), 14 -methyl pentadecenoic acid (iso C16:1 / iso hexadecenoic acid), hexadecanoic acid (C16:0), hexadecenoic acid (C16:1), 15-methyl hexadecanoic acid (iso C17:0/ iso heptadecanoic acid), heptadecanoic acid (C17:0), heptadecenoic acid C17:1 and/or 14-methyl hexadecanoic acid (anteiso C17:0/ anteiso heptadecanoic acid).

7. Composition according to any one of claims 1 to 6, wherein the total fatty acids of the composition are present in proportions as defined below:
- from 6.7 % to 7.7 % of iso C14:0,
- from 3.1 % to 4 % of C14:1,
- from 9.8 % to 11 % of anteiso C15:0,
- from 0.1 % to 0.7 % of C15:0,
- from 61.5 % to 62.5 % of iso C16:0,
- from 0.5 % to 1.5 % of iso C16:1,
- from 3.1 % to 4 % of C16:0,
- from 0.1 % to 7.7 % of C16:1,
- from 0.5 % to 1.5 % of C17:0,
- from 0.5 % to 1.5 % of C17:1,
- from 1.1 % to 2.1 % of iso C17:0,
and
- from 6.7 % to 7.7 % of anteiso C17:0,
the percentages being expressed by weight of the total fatty acids of the composition.

8. Composition according to any one of claims 1 to 7, which further comprises any other type of lipids and/or impurities, the lipids being free fatty acids, oligomers, sterols, fatty acid esters, sterol esters, monoacylglycerols, and/or diacylglycerols.

9. Microorganism extract comprising a lipid composition according to any one of claims 1 to 8.

10. Use of a composition according to any one of claims 1 to 8 or of an extract according to claim 9, for the preparation of biofuel, lubricant, surfactants, coatings, solvents, food ingredients or as synthesis intermediates for oleochemistry.

11. Pharmaceutical or cosmetic composition comprising a lipid composition according to any one of claims 1 to 8, or an extract according to claim 9, alone or in combination with a pharmaceutically or cosmetically acceptable carrier.

12. Cosmetic composition according to claim 11, in combination with a firming active substance, particularly in the form of foam.

13. Use of a cosmetic composition as defined in claim 12, for the styling of keratin fibres.

14. Lipid composition according to any one of claims 1 to 8, or extract according to claim 9, for use as a medicament.
